Europäisches Patentamt

European Patent Office

Office européen des brevets

(11) Publication number: **0 142 957**
**A2**

## (12) EUROPEAN PATENT APPLICATION

(21) Application number: **84307530.0**

(22) Date of filing: **01.11.84**

(51) Int. Cl.⁴: **A 61 F 9/02**

(30) Priority: **17.11.83 US 552999**

(43) Date of publication of application:
**29.05.85 Bulletin 85/22**

(84) Designated Contracting States:
**AT BE CH DE FR GB IT LI LU NL SE**

(71) Applicant: **Bell, Michael**
**P.O. Box 400**
**Warrington Pennsylvania 18976(US)**

(72) Inventor: **Bell, Michael**
**P.O. Box 400**
**Warrington Pennsylvania 18976(US)**

(74) Representative: **Shaw, Laurence**
**George House George Road**
**Edgbaston Birmingham B15 1PG(GB)**

(54) Eyewear for dusty environments.

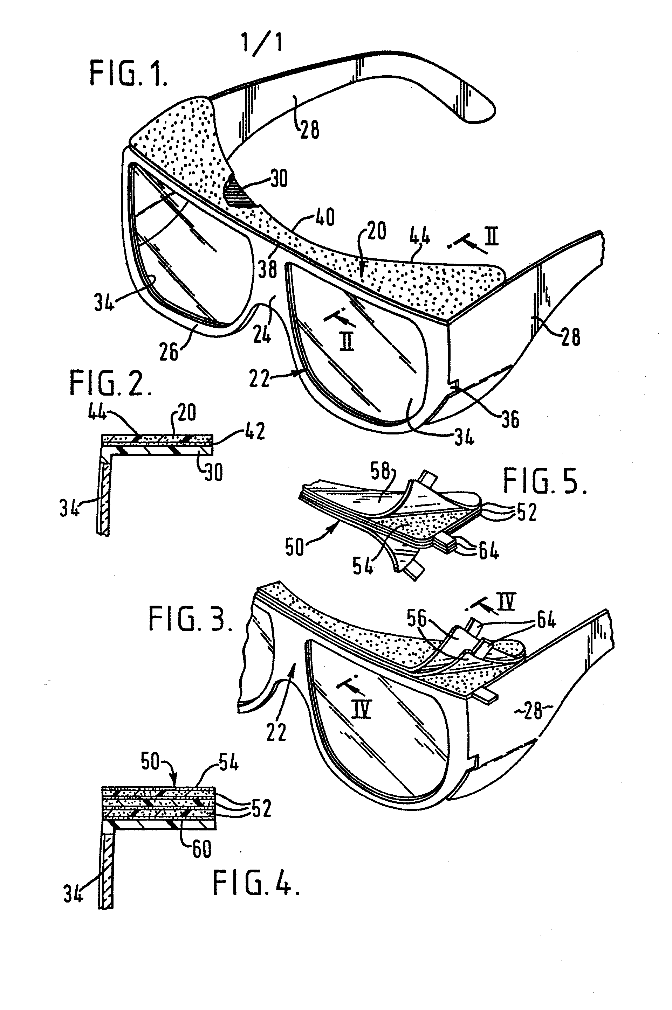

(57) Eyewear, such as safety glasses (22), includes means (20, 50) between the brow guard (30) and the brow of the wearer, the means being adapted to trap dust particles. For ferromagnetic particles the means (20) is a magnetic material, and for non-magnetic dust the means (50, Figure 3) has a tacky adhesive layer (54). Preferably a stack of layers (52) is provided, the layers being discarded when they are dust-laden.

FIG. 1.

EP 0 142 957 A2

Croydon Printing Company Ltd.

0142957

## EYEWEAR FOR DUSTY ENVIRONMENTS

This invention relates to eye wear for dusty environments and more particularly to eye protection means for use in combination with eye glasses, spectacles or goggles or like eye protection or eyewear.

Various industrial environments are characterised by a high concentration of air-borne dust particles. Such particles present a hazard to unprotected personnel. While safety goggles may provide some measure of protection against air-borne particles, the main advantage of such goggles is in protecting the eye from flying debris, etc. Air-borne particles, particularly of a fine size, may float around the periphery of the eye glasses or goggles and gain ingress to the eye.

The Fendall Company of Arlington Heights, Illinois has offered for sale safety glasses which include magnetic inserts on the temple pieces of the eye glass frame. The inserts are designed to attract dust particles and away from the wearer's eyes. In order to insure sufficient attraction of the magnetic particles from all areas around the eyes to the temples, relatively expensive magnets must be utilized. This is undesirable from the standpoint of cost, comfort, appearance, etc.

While air-borne magnetic particles represent a significant hazard to the eye, such particles form only a small portion of air-borne dust particles which can present a hazard. Many industrial environments have significant levels of non-ferrous air-borne dust particles. Examples of such environments are found in textile mills, lumber mills, etc. where cotton, wood or other non-ferrous dust may permeate the air.

It is one object of the invention to provide eye protection or eyewear, in which eye protection means is present to prevent or minimise the risk that airborne dust will reach the eye. It is another object to provide such items which may easily be freed of adherent dust.

According to one aspect of the invention there is provided a pair of glasses or eyewear, having eye protection means to attract dust particles characterised in that the eye protection means (20, 50) extends between the brow guard (30) and the brow of the wearer.

Preferably, the eye protection means is secured to the brow guard by adhesive and the brow guard extends from the front faces of the glasses and the brow of the wearer.

The invention provides one version adapted for use with ferromagnetic dust and another for use with non-magnetic dust.

In order that the invention may be well understood it will now be described with reference to the accompanying diagrammatic drawings in which:

Figure 1 is a perspective view of safety glasses according to the invention;

Figure 2 is an enlarged sectional view taken along line II-II of Figure 1;

Figure 3 is a perspective view of a portion of a second safety glasses of the invention;

Figure 4 is an enlarged sectional view taken along line IV-IV of Figure 3;

Figure 5 is a perspective view of a corner of the embodiment of Figure 3.

Where possible the same reference numerals are used in the description of the different embodiments.

A pair of safety goggles or glasses 22 comprise a plastic frame 24 having a front lens holding portion 26, a pair of
temple pieces 28 and an eye brow guard 30. The front
portion 26 includes a pair of openings in which a pair of
spectacle lenses 34 (either prescription or non-
prescription) are disposed. The temple pieces 28 are hinged
at 36 located at the sides of the front portion 26. The
brow guard 30 extends along the top edge 38 of the front
portion 26 and is shaped so that its peripheral edge 40 is
disposed immediately adjacent the brow of the wearer, not
shown.

The eye protection means comprises a thin flexible strip 20
formed of a plastic material having ferromagnetic
properties, cut to shape to cover the entire top surface of
the brow portion 30 and secured thereto by a lower pressure
sensitive adhesive surface 42. The magnetic flexible strip
comprises a synthetic rubber such as neoprene, impregnated
with barium ferrites or ferric oxides and then magnetised in
a multipole arrangement.

When the glasses of Figure 1 are worn, air-borne
ferromagnetic particles which would tend to ingress to the
eye from the space between the brow guard and the brow of
the wearer will be attracted to and retained on the top
surface 44 of the eye protection means 20. The adhered
particles may be wiped off when required.

The embodiment of Figure 3 is intended for use where there is no likelihood of magnetic dust. The eye protection means 50 comprises a stack of dust trapping layers 52 each shaped to completely cover the brow guard 30. Each layer 52 has an upper surface 54 and a lower surface 56, the upper surface 54 of each layer carrying an adhesive by which the layers are held together. A peelable protective cover sheet 58 is disposed on the top surface 54 of the uppermost layer of the stack and an adhesive layer 60, Figure 4, is present on the underside 56 of the bottom layer to secure the stack to the brow guard 30.

Each layer 52 is formed of a very thin, flexible material, such as paper stock or Mylar, (Registered Trade Mark) so that the stack can include multiple layers, e.g. up to ten, and yet be quite thin and lightweight.

The stack is stored with a temporary protective cover sheet 62, similar to cover sheet 58, on the undersurface 60 of the bottom layer of the stack.

The cover sheets 58 and 62 serve to protect the outer adhesive layers until the stack 50 is ready for mounting and use on the glasses 22. Each layer of the stack and the two cover sheets includes projecting a finger grasping tab 64 to enable a layer to be peeled away.  In use the bottom cover sheet 62 is peeled off by grasping its tab portion 64, thereby exposing the underside adhesive layer 60; the stack is then secured on the brow guard 30 by the underside adhesive 60. When the glasses are ready to be worn in a dusty environment the protective cover sheet 58 is peeled off the top layer 52 to expose the uppermost pressure sensitive acrylic based adhesive surface 54 when then acts to trap any dust particles which otherwise would have landed on the brow guard and reached the eye. After the top layer has been loaded with dust particles the user then peels the top layer 52 off of the stack 50 by tab 64, thereby exposing the fresh adhesive layer 54 of the underlying layer 52. The process is repeated until the lowermost layer is full whereupon it is peeled off and a new stack 52 is secured to the brow guard 30.

The eye protection means of the invention are simple in construction, can be made at low cost and offer a high degree of protection to the eye by trapping various types of air-borne dust particles.

## CLAIMS

1. A pair of glasses or like eyewear, having eye protection means to attract dust particles characterised in that the eye protection means (20, 50) extends between the brow guard (30) and the brow of the wearer.

2. A pair of glasses according to Claim 1 characterised in that the eye protection means (20, 50) is secured by adhesive to the brow guard (30) which is shaped and dimensioned to extend from the front face (26) of the glasses (22) and the brow of the wearer.

3. A pair of glasses according to Claim 1 or 2 characterised in that the eye protection means (20) comprises magnetic material for trapping ferromagnetic dust particles.

4. A pair of glasses according to Claim 1 or 2 characterised in that the eye protection means (50) has an upper surface (52) covered by tacky adhesive to trap dust particles falling thereon.

5. A pair of glasses according to Claims 3 or 4 characterised in that the eye protection means (50) comprises a stack of layers (52) each having an upper surface (54) covered by pressure sensitive adhesive.

6. A pair of glasses according to Claim 5 characterised by tab means (64) to enable the uppermost layer to be removed when it is laden with dust particles.

7. Eye protection means (50) for use in glasses according to Claims 4, 5, 6 characterised in that the means (50) comprises layers of material each having an upper surface covered by adhesive, tab means (64) being present to facilitate separation of the layers, peelable cover sheets (58, 60) being present on the centremost top and bottom layers.

0142957

FIG.1.    1/1

FIG.2.

FIG.5.

FIG.3.

FIG.4.